Europäisches Patentamt

European Patent Office

Office européen des brevets

(19)

(11) Veröffentlichungsnummer: **0 358 990**

**A1**

## (12) EUROPÄISCHE PATENTANMELDUNG

(21) Anmeldenummer: 89115554.1

(22) Anmeldetag: 23.08.89

(51) Int. Cl.5 **A61F 9/00 , A61B 17/32**

(30) Priorität: 25.08.88 DE 3828787

(43) Veröffentlichungstag der Anmeldung:
**21.03.90 Patentblatt 90/12**

(84) Benannte Vertragsstaaten:
**AT BE CH DE ES FR GB GR IT LI LU NL SE**

(71) Anmelder: **ADATOMED PHARMAZEUTISCHE UND MEDIZIN-TECHNISCHE GESELLSCHAFT MBH**
**Am Moosfeld 27**
**D-8000 München 82(DE)**

(72) Erfinder: **Klemmen, Ulrich, Dr.med.**
**Eybner Strasse 4**
**A-3100 St. Pölten(AT)**

(74) Vertreter: **Nöth, Heinz, Dipl.-Phys. et al**
**Patentanwälte Pfenning, Meinig & Partner**
**Mozartstrasse 17**
**D-8000 München 2(DE)**

(54) **Vorrichtung zur Durchführung einer Trepanation am Auge.**

(57) Eine Vorrichtung zur Durchführung einer Trepanation am Auge mit einem an den zu schneidenden Augenteil heranführbaren, insbesondere in die Augenvorderkammer einschiebbaren Trepan, der für die Schnittbewegung in einem Haltering (2) drehbar und gleichzeitig in Richtung der Ringachse bewegbar geführt ist, und durch eine Rückholfeder in Richtung zu seiner Ausgangslage im Haltering (2) vorgespannt ist.

FIG. 1

EP 0 358 990 A1

## Vorrichtung zur Durchführung einer Trepanation am Auge

Die Erfindung betrifft eine Vorrichtung zur Durchführung einer Trepanation am Auge mit einem an den zu schneidenden Augenteil heranführbaren. insbesondere in die Augenvorderkammer einschiebbaren Trepan.

Bei am Auge durchzuführenden Schnitten im Verlauf von Operationen ist eine einwandfreie Führung des Schnittwerkzeugs bzw. Trepans erforderlich. Beispielsweise bei der operativen Beseitigung einer infolge Katarakt getrübten Linse ist zunächst in der Linsenkapsel ein Schnitt anzubringen, um den Linsenkörper zu entfernen und gegebenenfalls durch eine künstliche Augenlinse - eine sogenannte Intraokularlinse - zu ersetzen. Hierzu ist es erforderlich, in dem vorderen Kapsellamellenteil einen einwandfreien Schnitt anzubringen. Falls die vordere Kapsellamelle kreisrund ausgeschnitten werden soll, ist zudem eine exakte Zentrierung des Trepans in der Augenvorderkammer erforderlich.

Aufgabe der Erfindung ist es, eine Vorrichtung der eingangs genannten Art zu schaffen, mit der eine einwandfreie Anordnung des Trepans gegenüber dem zu schneidenden Teil am Auge, insbesondere eine einwandfreie zentrische Anordnung, in der Augenvorderkammer für eine nachfolgende Kapsulotomie erreicht wird.

Diese Aufgabe wird erfindungsgemäß dadurch gelöst, daß der Trepan für die Schnittbewegung in einem Haltering drehbar und dabei gleichzeitig in Richtung der Ringachse bewegbar geführt ist, und daß der Trepan durch eine Rückholfeder in Richtung zur Ausgangslage im Haltering vorgespannt ist.

Dadurch, daß der Trepan für die Schnittbewegung in einem Haltering geführt ist, wird eine genaue Positionierung und Zentrierung des Trepans gegenüber dem zu schneidenden Augenteil erreicht. Insbesondere wird dies erreicht beim Einführen des Halterings in die Augenvorderkammer, wobei gleichzeitig eine genaue Zentrierung gegenüber der Vorderkapsel der Augenlinse erreicht wird.

Der Trepan wird bevorzugt an der Innenseite des Halterings geführt. Hierzu kann der Trepan zylinderförmig ausgebildet sein und bevorzugt eine kreisringförmige Schneidkante an seiner Unterseite besitzen.

Der Trepan läßt sich mit Hilfe einer Gewindeführung (Außengewinde an der Mantelfläche des zylinderförmigen Trepans und Innengewinde an der Innenfläche des Halterings) führen.

Durch eine relativ geringe Rotation des Trepans (etwa um 90° oder auch weniger) wird der Trepan in Richtung der Ringachse abgesenkt. Durch dieses Verdrehen und Absenken des Trepans wird durch die ringförmige Schneidkante an der Unterseite des Trepans ein einwandfreier Schnitt in der vorderen Linsenkapsel ausgeführt.

Zur Erzielung der Drehbewegung kann ein extern betätigbares Zugkabel vorgesehen sein, das über ein im Haltering geführtes Mitnehmerelement durch Einrasten mit dem Trepan verbunden ist.

Der Trepan wird nach Durchführung der Schnittbewegung durch eine Rückholfeder, welche bevorzugt als Zugfeder ausgebildet ist, die mit dem einen Ende am Haltering fixiert ist und mit dem anderen Ende am Mitnehmerelement angreift, in seine Ausgangsposition vor der Schnittbewegung zurück gebracht. Es ist auf diese Weise möglich, auch mehrere Schnittbewegungen nacheinander auszuführen.

Der Haltering besitzt in bevorzugter Weise in einem oben liegenden Querschnittsteil eine ringförmige Infusionskammer mit nach oben geöffneten Infusionslöchern. Auf diese Weise ist es möglich, daß bei in die Vorderkammer eingesetztem Haltering die Linsenkammer tief gehalten wird und ein gleichmäßiger Druck auf die vordere Linsenkammer ausgeübt wird. Hierzu kann die Infusionskammer mit einem externen Infusionsrohr verbunden sein, durch welches der Infusionskammer das erforderliche Infusionsmedium zugeführt wird.

In einem unten liegenden Querschnittsteil des Halterings wird das mit dem Trepan verbundene Mitnehmerelement geführt. An diesem greift ein extern betätigbares Zugkabel an.

In bevorzugter Weise können das externe Infusionsrohr und das externe Zugkabel in einer Einheit, die als Führungsstift ausgebildet ist, untergebracht sein.

Mit Hilfe des Führungsstifts läßt sich der Haltering mit dem Trepan am bzw. im Auge - und hier insbesondere in der vorderen Linsenkammer - einwandfrei positionieren und nach Durchführung des Schnittes wieder entfernen. Am Führungsstift können ferner die zur Betätigung des Trepans erforderlichen Handgriffe, welche über den Seilzug und den Wagen auf den Trepan wirken, vorgesehen sein. Ferner können entsprechende Führungsringe, durch welche zur Führung des Führungsstiftes Finger bei der Handbetätigung hindurchgesteckt werden können, am Führungsstift befestigt sein.

Der Haltering besteht bevorzugt aus einem sterilisierbaren Material, so daß er mehrfach verwendbar ist.

Anhand der Figuren wird die Erfindung noch näher erläutert. Es zeigt:

Fig. 1 eine Gesamtansicht einer Vorrichtung zur Durchführung einer Trepanation als Ausführungsbeispiel der Erfindung in Draufsicht;

Fig. 2 in Seitenansicht die in der Fig. 1 dargestellte Vorrichtung mit aus dem Haltering gelöstem Trepan;

Fig. 3 eine für das Ausführungsbeispiel der Fig. 1 anwenbare Ausführungsform eines Halterings, wobei in der Fig. (a) der Trepan sich in der Ausgangsstellung befindet, und in der Fig. (b) der Trepan sich in einer Position nach Vollendung einer Schnittbewegung befindet; und

Fig. 4 eine Draufsicht in teilweise geschnittener Darstellung des in der Fig. 3 dargestellten Halteringes mit eingesetztem Trepan.

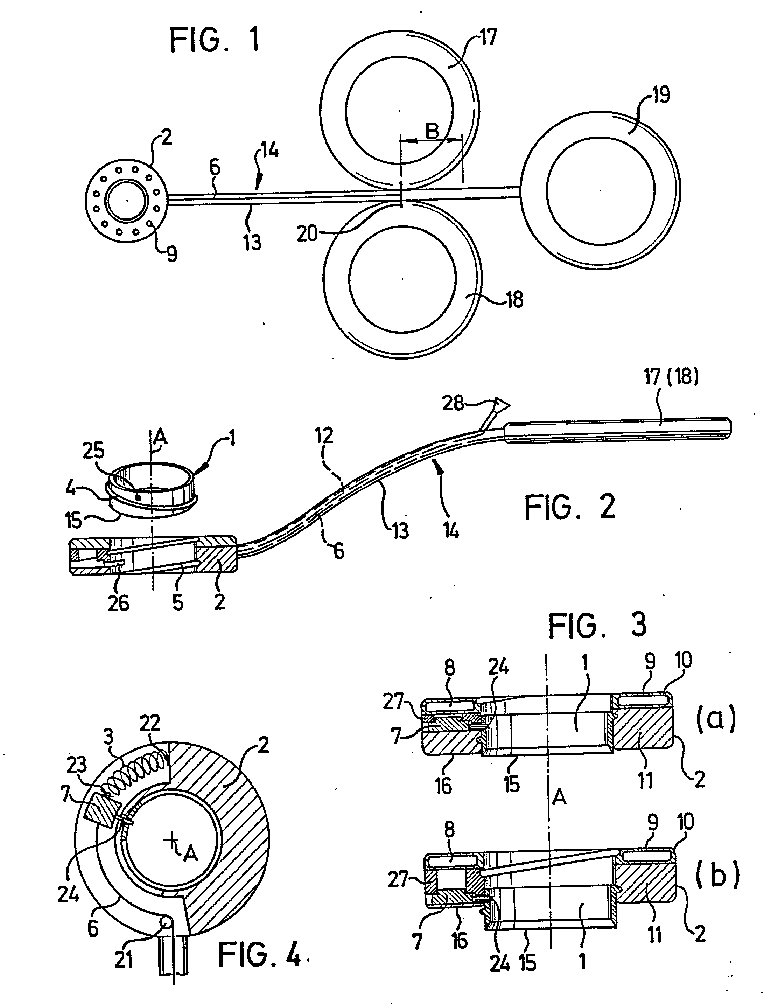

Das in den Figuren 1 bis 4 dargestellte Ausführungsbeispiel einer Vorrichtung zur Durchführung einer Trepanation, insbesondere einer Kapsulotomie, besitzt einen Haltering 2, in den ein Trepan 1 einsetzbar ist. Der Trepan 1 besitzt ein Außengewinde 4, welches in ein Innengewinde 5 des Halterings 2 einschraubbar ist. Der Haltering 2 ist an einem Führungsstift 14 befestigt. Der Führungsstift 14 besitzt an dem dem Haltering 2 entgegengesetzt liegenden Ende drei ringförmige Fingergriffe 17, 18 und 19. Mit Hilfe des Führungsstiftes 14 läßt sich der Haltering 2 mit dem im Ringinnern gelagerten Trepan 1 in eine Augenvorderkammer einführen und exakt zentrieren. Der Gesamtdurchmesser des Halterings 2 beträgt 8 mm. Für das Einfügen des Halteringes 2 in die Augenvorderkammer genügt mithin eine 9 mm lange Öffnung in der Augencornea.

Der Haltering 2 besitzt, wie aus den Figuren 2 und 3 zu ersehen ist, einen etwa quadratischen Querschnitt mit maximal 1 mm² und abgerundeten Kanten. Zur Führung des Trepans 1 ist die Innenseite des Halterings 2 mit einem Innengewinde 5 versehen. In dieses Innengewinde ist ein Außengewinde 4 am zylindrisch ausgebildeten Trepan 1 einschraubbar. Auf diese Weise wird erreicht, daß beim Drehen des Trepan 1 gleichzeitig eine axiale Verschiebung in Richtung der Ringachse A erreicht wird.

Die Drehbewegung des Trepans 1 wird über ein Zugkabel 6, das als dünner Draht oder entsprechend stabiler Kunststofffaden ausgebildet sein kann, extern bewirkt. Hierzu werden am Führungsstift 14 die beiden Fingergriffe 17 und 18 in der einen Richtung eines Doppelpfeiles B zum Fingergriff 19 hin bewegt. Das Zugkabel 6 ist an einer Befestigungsstelle 20 mit den beiden Fingergriffen 17 und 18 verbunden. Über eine im Haltegriffinnern vorgesehene Umlenkstelle 21 wird das Zugkabel 6 zu einem Wagen 7 geführt, der Bestandteil eines Mitnehmerelements für den Trepan 1 bei dessen Schneidbewegung ist. Am Wagen 7 greift eine Rückholfeder 3 an. Diese Rückholfeder 3 ist als Zugfeder ausgebildet und mit ihrem einen Ende 22 am Haltering 2 fixiert und greift mit ihrem anderen Ende 23 am Wagen 7 an. Der Wagen 7 besitzt als weiteren Bestandteil des Mitnehmerelements einen Mitnehmerstift 24, der in eine entsprechende Mitnehmeröffnung 25 am Trepan federnd einrastbar ist. Der Mitnehmerstift 24 steht hierzu beispielsweise unter einer Federkraft, die ihn in Richtung auf die Ringachse A zu schiebt. Gegen diese Federkraft kann der Mitnehmerstift 24 im Wagen 7 von der Ringachse A wegbewegt werden, so daß der Trepan 1 ungehindert in den Haltering 2 eingeschraubt werden kann. Bei dieser Ausführungsform ist der Trepan 1 auswechselbar in den Haltering 2 einsetzbar. Der Mitnehmerstift 24 ragt dabei durch eine in Richtung des Innengewindes 5 verlaufende Öffnung 26, die schlitzartig ausgebildet ist, so daß der Mitnehmerstift 24 beim Anziehen an das Zugkabel 6 zusammen mit dem Wagen 7 entgegen der Kraft der Rückholfeder 3 mitbewegt werden kann. Dabei nimmt der Mitnehmerstift 24, welcher durch die Mitnehmeröffnung 25 des eingesetzten Trepans 1 ragt, den Trepan mit.

Bei der Drehbewegung entgegen der Kraft der Rückholfeder 3 bewegt sich der Trepan aus der in der Fig. 3(a) gezeigten Ausgangsstellung in axialer Richtung entlang der Ringachse A nach unten in die in der Fig. 3(b) gezeigte Endstellung. Bei dieser Schnittbewegung wird der gewünschte Schnitt beispielsweise in der vorderen Linsenkapsellamelle durchgeführt. Nach vollendeter Kapsulotomie, d. h. bei frei schwebender Kapsellamelle, wird der Trepan 1 wieder in seine Ausgangslage (Fig. 3(a)) aufgrund der Kraft der Rückholfeder 3 zurückgebracht. Dabei werden auch die beiden Fingergriffe 17 und 18 in der anderen Richtung des Doppelpfeiles B vom Fingergriff 19 weg bewegt und in ihre Ausgangslage zurückgebracht. Es kann dann eine erneute Schnittbewegung durchgeführt werden.

Wie insbesondere aus der Fig. 3 zu ersehen ist, befindet sich der Wagen 7 mit dem Mitnehmerstift 24 in einem unteren Querschnitt 11 des Halteringes 2. Auch die Rückholfeder 3 sowie das Zugkabel 6 und auch die Umlenkstelle 21 sind in diesem unteren Querschnitt 11 des Halteringes 2 angeordnet. Für den Wagen 7 ist in diesem unteren Querschnittsteil 11 eine Schienenführung 27 vorgesehen. Diese Schienenführung gewährleistet, daß der Wagen 7 in Richtung des Innengewindes 5 und des Außengewindes 4 geführt wird. Auf diese Weise wird über den durch die Öffnung 26 ragenden Mitnehmerstift 24 eine einwandfreie Mitführung des Trepans 1 bei der Schnittrotationsbewegung gewährleistet. Wie die Fig. 3 zeigt, befindet sich in der Ausgangslage der Wagen 7 und auch der Trepan 1 in einer oberen Stellung, bei welcher die Schnittkante 15 am Trepan 1 etwa 0,1 bis 0,2 mm über einer unteren Begrenzungsfläche 16 des Halterings 2 liegt. Nach Durchführung der Schnittrotationsbewegung hat sich der Trepan 1 mit seiner Schnittkante 15 nach unten bewegt, und die Schnittkante 15 liegt in der in der Fig. 3(b) gezeigten Position etwa 0,5 mm unterhalb der unteren Begrenzungsfläche 16 des Halterings 2. Der Trepan 1 besitzt mithin eine Mindesthöhe von 0,8 mm. Die axiale Bewegung des Trepans während der Drehung beträgt etwa 0,6 bis 0,7 mm. Man ist bestrebt, mit einer möglichst geringen Rotationsbewegung (etwa 90°) auszukommen. Entsprechend werden das Außengewinde 4 am Trepan 1 und das Innengewinde 5 am Haltering 2 bemessen.

In einem oberen Querschnittsteil 10 (Fig. 3) befindet sich eine ringförmige Infusionskammer 8. Diese Infusionskammer ist mit nach oben gerichteten Infusionslöchern 9 ausgestattet. Die Infusionskammer 8 steht mit einem externen Infusionsrohr 12 in Strömungsverbindung, so daß von außen her ein Infusionsmedium in die Infusionskammer 8 eingebracht werden kann und von dort durch die Infusionslöcher 9 ausströmen kann. Auf diese Weise läßt sich bei einem in die Vorderkammer eines Auges eingesetzten Haltering die Kammer tief halten und ein gleichmäßiger Druck auf die vordere Linsenkapsel ausüben.

Wie die Fig. 2 zeigt, können das externe Infusionsrohr 12 und eine Zugkabelführung 13 zu einer Einheit, nämlich zu dem Führungsstift 14, verbunden sein. Mit 28 (Fig. 2) ist ein Einlaß für das Infusionsmedium bezeichnet.

**Ansprüche**

1. Vorrichtung zur Durchführung einer Trepanation am Auge mit einem an dem zu schneidenden Augenteil heranführbaren, insbesondere in die Augenvorderkammer einschiebbaren Trepan, dadurch gekennzeichnet, daß der Trepan (1) für die Schnittbewegung in einem Haltering (2) drehbar und dabei gleichzeitig in Richtung der Ringachse (1) bewegbar geführt ist, und daß der Trepan (1) durch eine Rückholfeder (3) in Richtung zu seiner Ausgangslage im Haltering (2) vorgespannt ist.

2. Vorrichtung nach Anspruch 1, dadurch gekennzeichnet, daß der Trepan (1) im Innenraum des Halterings (2) geführt ist.

3. Vorrichtung nach Anspruch 1 oder 2, dadurch gekennzeichnet, daß der Trepan (1) mittels einer Gewindeführung (4, 5) am Haltering (2) geführt ist.

4. Vorrichtung nach einem der Ansprüche 1 bis 3, dadurch gekennzeichnet, daß der Trepan (1) um die Ringachse (A) um etwa 90° verdrehbar und dabei um etwa 0,6 mm in Richtung der Ringachse (A) verschiebbar am Haltering (2) geführt ist.

5. Vorrichtung nach einem der Ansprüche 1 bis 4, dadurch gekennzeichnet, daß am Trepan (1) ein extern betätigbares Zugkabel (6), welches über den Haltering (2) geführt ist, angreift.

6. Vorrichtung nach einem der Ansprüche 1 bis 5, dadurch gekennzeichnet, daß das Zugkabel (6) über ein am Haltering (2) geführtes Mitnehmerelement (7, 24), das am Trepan (1) einrastbar ist, am Trepan angreift.

7. Vorrichtung nach einem der Ansprüche 1 bis 6, dadurch gekennzeichnet, daß die Rückholfeder (3) als Zugfeder ausgebildet ist, die mit dem einen Ende am Haltering (2) fixiert ist und mit dem anderen Ende am Mitnehmerelement (7, 24) angreift.

8. Vorrichtung nach einem der Ansprüche 1 bis 6, dadurch gekennzeichnet, daß der Haltering (2) einen oben liegenden Querschnittsteil (10), der eine ringförmige Infusionskammer (8) mit nach oben geöffneten Infusionslöchern (9) aufnimmt, und einen unten liegenden Querschnittsteil (11), in welchem das mit dem Trepan (1) verbundene Mitnehmerelement (7, 24) und das Zugkabel (6) geführt sind, aufweist.

9. Vorrichtung nach Anspruch 8, dadurch gekennzeichnet, daß die Infusionskammer (8) mit einem externen Infusionsrohr (12) verbunden ist.

10. Vorrichtung nach einem der Ansprüche 1 bis 9, dadurch gekennzeichnet, daß das externe Infusionsrohr (12) mit einer externen Zugkabelführung (12), an welcher der Haltering (2) befestigt ist, zu einer Einheit (Führungsstift 14) verbunden sind.

11. Vorrichtung nach einem der Ansprüche 1 bis 10, dadurch gekennzeichnet, daß sich eine Schneidkante (15) des Trepans (1) in der Ausgangslage etwa 0,1 bis 0,2 mm oberhalb einer unteren Begrenzungsfläche (16) des Halterings (2) befindet.

12. Vorrichtung nach einem der Ansprüche 1 bis 11, dadurch gekennzeichnet, daß der Trepan (1) zylinderförmig ausgebildet ist und auf seiner Mantelfläche ein Außengewinde (4) aufweist, das in ein Innengewinde (5) des Halterings (2) eingreift.

EP 0 358 990 A1

FIG. 1

FIG. 2

FIG. 3

FIG. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 038 024  (SCHLEGEL)<br>* Insgesamt *<br>--- | 1,2,5 | A 61 F    9/00<br>A 61 B   17/32 |
| A | FR-A-2 588 751  (IMBERT)<br>--- | | |
| A | GB-A-  591 548  (RAYMONT)<br>--- | | |
| P,A | US-A-4 766 897  (SMIRMAUL)<br>* Zusammenfassung; Spalte 2, Zeile 49 -<br>Spalte 3, Zeile 10; Figuren *<br>----- | 1,2,5-7<br>,12 | |
| | | | **RECHERCHIERTE SACHGEBIETE (Int. Cl.5)**<br><br>A 61 F<br>A 61 B |

Der vorliegende Recherchenbericht wurde für alle Patentansprüche erstellt

| Recherchenort | Abschlußdatum der Recherche | Prüfer |
|---|---|---|
| DEN HAAG | 21-11-1989 | STEENBAKKER J. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes
    Dokument